# EUROPEAN PATENT SPECIFICATION

(11) **EP 2 349 257 B1**
(45) Date of publication and mention of the grant of the patent: **27.11.2013**
(21) Application number: 08772747.5
(22) Date of filing: 17.06.2008
(51) Int. Cl.: A61K 31/4196, A61K 31/4188, A61P 31/12, A61P 43/00

(54) **USE OF PAF RECEPTOR FOR TREATING INFECTIONS CAUSED BY FLAVIVIRIDAE**
VERWENDUNG DES PAF-REZEPTORS ZUR BEHANDLUNG VON DURCH FLAVIVIRIDAE VERURSACHTEN INFEKTIONEN
UTILISATION DU RECEPTEUR DU FACTEUR D'ACTIVATION PLAQUETTAIRE (PAF) DANS LE TRAITEMENT DES INFECTIONS CAUSEES PAR FLAVIVIRIDAE

(43) Date of publication of application: 03.08.2011
(73) Proprietor: Universidade Federal de Minas Gerais-UFMG, CEP-31270-901 Belo Horizonte, MG (BR)
(72) Inventor: TEIXEIRA, Mauro Martins, Minas Gerais - CEP 31275-030 (BR); DE SOUZA, Daniele da Glória, Minas Gerais - CEP 30840-490 (BR)
(74) Representative: Kaiser, Jürgen
(86) International application number: PCT/BR2008/000182
(87) International publication number: WO 2009/152589

(56) References cited:
- WO-A2-03/072135
- WO-A2-03/082199
- JP-A- 7 304 689
- ANTHONY J FREEMAN ET AL: "Immunopathogenesis of hepatitis C virus infection", IMMUNOLOGY AND CELL BIOLOGY, vol. 79, no. 6, 30 December 2001 (2001-12-30), pages 515-536, XP55021293, ISSN: 0818-9641, DOI: 10.1046/j.1440-1711.2001.01036.x
- D. G. SOUZA ET AL: "Essential role of platelet-activating factor receptor in the pathogenesis of Dengue virus infection", PROCEEDINGS OF THE NATIONAL ACADEMY OF SCIENCES, vol. 106, no. 33, 18 August 2009 (2009-08-18), pages 14138-14143, XP55021310, ISSN: 0027-8424, DOI: 10.1073/pnas.0906467106

## Description

The present invention relates to a use of a platelet activating factor (PAF) receptor anatagonist for the preparation of a medicament for treating infectious diseases caused by viruses of the *Flaviviridae* family.

This invention brings forth a new drug of an already known product used in medical assistance for treating diseases caused by virus of the *Flaviviridae* family, mainly for treating Dengue and Hemorrhagic Dengue Fever (HDF).

### Background of the Invention

Dengue is caused by a virus of the *Flaviviridae* family of the enveloped viruses. Flaviviruses are important human pathogens, which are prevalent throughout the world. There are at least 39 flaviviruses associated with human diseases, including dengue and yellow fever. Related pathologies cause hemorrhagic, shock, encephalitic and fever diseases.
As for dengue specifically, there are four serotypes and the genome is constituted by a single strand of RNA of about 11,000 bases. The translated genome brings about a large polyprotein whose co-translational processing by viral enzymes and host gives birth to three structural proteins (C, M and E proteins) and seven nonstructural proteins (NS1, NS2a, NS2b, NS3, NS4a, NS4b e NS5) involved in viral replication (Chambers TJ, Hahn CS, Galler, R, Rice, CM. (1990). Flavivirus genome organization, expression and replication. Annu. Rev. Microbiol., 44: 649-688). Humans are the major hosts of the dengue virus which is mainly transmitted through the bites of infective female mosquitoes of the genus *Aedes, A. aegypti* (Mukhopadhyay S, Kuhn RJ, Rossmann MG. (2005). A structural perspective of the flavivirus life cycle. Nat Rev Microbiol. 3, 13-22).

There are two most relevant forms of dengue manifestation, the classical form and the hemorrhagic form. Convalescence may last for several weeks during which period manifestations of asthenia are presented by the patient. Classic Dengue fever may be asymptomatic or patients may present with symptoms including high fever, headache, retro-orbital pain, myalgia, arthralgia, nausea and vomiting, diarrhea, postural hypotension, abdominal pain, lumbar pain and pain in lower limbs. Classic dengue is known popularly as "break-bone fever". Additionally, skin and mucous membrane alterations, such as petechae, may occur. Even in case of the classical form, hemorrhage, such as epistaxis, bleeding gums and digestive hemorrhage may appear. The classical form usually lasts for 6 to 8 days (data from the State Health Secretariat of Minas Gerais).
Dengue hemorrhagic fever and Dengue shock syndrome are the most severe presentations of Dengue infection and initially present the same symptoms of the classical form. Hemorrhagic phenomena occur in the second or third days of disease and are mainly manifested as cutaneous and digestive tract bleeding; metrorrhagia, epistaxis, bleeding gums and bleedings at other sites may also occur. Endothelial cell injury and activation of leukocytes and platelets secondary to the infection form the physiopathologic basis of hemorrhagic dengue. Activation of leukocytes, platelets and endothelial cells lead to thrombocytopenia and increased vascular permeability. The latter leads to increased hematocrit and decreased blood pressure and hypovolemic shock. The major problems associated with severe dengue - shock and hemorrhagic manifestations - are, hence, a consequence of excessive activation of the innate immune system during the infection (Halstead, SB. (2003). Antibody, macrophages, dengue virus infection, shock and hemorrhage: a pathogenic cascade. Rev. Infect. Dis. 11:S830-S839) (Rothman A.L. (2003). Immunology and immunopathogenesis of dengue disease. Adv. Virus Res. 60:397-419).

Dengue disease is now the most important arbovirosis affecting humans and represents a severe public health problem in the world. According to estimates from The World Health Organization (WHO), 50 to 100 million people are infected annually in more than 100 countries in all continents, except for Europe. Approximately 550 thousand patients need to be hospitalized and 20 thousand die as a consequence of the dengue.

The favorable socioenvironmental conditions for the expansion of *Aedes aegypti* in Brazil have made it possible for the vector's spread as well as the advance of the disease, since it was reintroduced in the country in 1976. Its reintroduction was not controlled adequately by the traditionally employed methods of chemical combat to vector transmissible diseases in the country and the continent. Then in 1996, the Brazilian Ministry of Health reviewed its policy strategy and proposed an eradication plan called *Programa de Erradicação do Aedes Aegypti* - PEAa *(Aedes Aegypti's eradication program).* While implementing the plan, the mosquito's eradication proved to be technically infeasible in the short and medium run, as PEAa was absolutely incapable of responding to the epidemiologic complexity of the disease.

Results obtained in Brazil and in other countries - which show no evidence eradication of the vector in the short run- have led the Ministry of Health to proceed a new assessment of the advances and hindrances of such a policy, in order to establish a new program that might incorporate other elements, such as social mobilization and community commitment, which are judged indispensable for an adequate combat to a highly home-based vector.

In the 1990s, the serotype 3 was introduced in the country, which was swiftly disseminated through eight Brazilian states within a period of only three months. A high epidemic risk, with an increased incidence of hemorrhagic dengue fever (HDF), showed how easily the new serotypes and virus species can circulate with the daily-shifting populations.

The technical state of the art is able to offer control technologies of the vector - which also transmits the yellow fever - as well as treatment therapies for the symptoms associated with dengue fever.

Brazilian patent PI023907-9 presents a monitoring system of the mosquito transmitting dengue that uses traps, software, palmtops for capturing and analyzing insects in risk areas. Within the trap - called *mosquitrap -* the *atraedes,* a synthetic odor-releasing substance, is put in the bottom of a black vase filled with some water that imitates a mosquito nursery so as to attract and make female mosquitoes lay their eggs. When attracted by *Atraedes,* the insects enter the trap and are kept by an adhesive card put onto the wall of the vase. A week later, captured mosquitoes are counted by health agents who have been trained to identify the *Aedes Aegypti* in its adult form. There is no need for laboratory identification of insects.

WO2004/037272 describes a drug intended to act as an inhibitor of dengue infection, which is characterized by a carbohydrate molecule as an active component whose essential constituent is an oligosaccharide chain.

WO2005/069717 presents a treatment technology for dengue hemorrhagic fever that immediately resulted in increased platelets. US 6,696,281 describes vaccines against flaviviruses contain an attenuated virus.

In WO 2003/049672 methods and compounds are described for the treatment of illnesses mediated by flaviviruses. WO 2004/084796 presents components for the treatment of flaviviridae infection as well as a method for its treatment. US 6,172,053 reports a therapeutic compound and formulation for treating viral diseases.

US 7,148,248 describes a method for treating or inhibiting the development of inflammation or sepsis. This technology is intended for using leukotriene C4 and D4 antagonist for treating or inhibiting inflammation or sepsis by increasing capillary permeability and spillover of white cells in the blood.

US 7,151,082 presents a HMG1 antagonist for treating inflammatory conditions. This technology comprises a pharmaceutical compound and a sepsis treatment method, including septic shock and acute respiratory distress syndrome as well as a method for monitoring or diagnosis of severity or potential lethality of sepsis.

Clinical actions for cases of dengue may be summarized in a sequence as follows: i) patients submitted to positive capillary fragility test and/or other light hemorrhagic manifestations are assisted in laboratories and sent for platelet count; ii) increased hematocrit and/or decreased platelet count detected (< or equal to 100.000/mm³); treatment comprises parenteral hydration associated to administration of dipirone (used in Brazil only) and paracetamol, continuous evaluation for any sign of worsening, clinical evaluation at least 6 every hours and laboratory tests (HT, platelet count, thorax X-ray) if necessary; c) patients showing signs of hypotension, loss of consciousness or shock are given ambulatory assistance with parenteral rehydration and specific serological analysis.

As there is no specific treatment for dengue, the protocol for medical assistance is restricted to detection of pain and fever, ambulatory assistance for hydration and analgesia accompanied by notification to *Vigilância Epidemiológica* Distrital (district epidemiologic vigilance agencies).

The development of specific treatment for dengue has been hindered by lack of knowledge on the disease pathogenesis. As a matter of fact, the major mechanism through which such viral infection leads to the hemorrhagic form of the disease is controversial. Contrasting hypotheses have attributed the severity of the illness to both viral factors and host factors (Rosen, L. (1997). The Emperor's New Clothes revisited, or the reflections on the pathogenesis of dengue hemorrhagic fever. Am. J. Trop. Med. Hyg. 26:337-343. Halstead, S.B. (1979). In vivo enhancement of dengue virus infection in rhesus monkeys by passively transferred antibody. J. Infect. Dis. 140:527-533; Rothman A.L. (2003). Immunology and immunopathogenesis of dengue disease. Adv. Virus Res. 60:397-419).

Recent studies in Peru and Sri Lanka have shown an association of the hemorrhagic form to viral genotypes. Some specific genetic determinants explaining such association were mapped (Watts DM, Porter KR, Putvatana P, Vasquez B, Calampa C, Hayes CG, Halstead SB. (1999). Failure of secondary infection with American genotype dengue 2 to cause dengue hemorrhagic fever. Lancet. 354:1431-1434; Messer, WB, Gubler, DJ. Harris, E., Sivananthan, K., da Silva, A.M. (2003). Emergence and global spread of dengue serotype 3, subset III virus. Emerg. Infect. Dis. 9:800-809; Messer WB, Vitarana UT, Sivananthan K, Elvtigala J, Preethimala LD, Ramesh R, Withana N, Gubler DJ, De Silva AM. (2002). Epidemiology of dengue in Sri Lanka before and after the emergence of epidemic dengue hemorrhagic fever. Am. J. Trop. Med. Hyg. 66:765-773).

However, proving that such genetic elements or other mechanisms are truly responsible for dengue virulence has shown to be difficult. Epidemiological studies have found an association between increased risk of manifestation of dengue hemorrhagic fever and a secondary infection by another strain of dengue virus (Halstead *et al.,* 1979; Guzman *et al.,* 1990, Rothman A.L. (2003).

This epidemiological finding suggests that the host immune response plays an important pathophysiological role in determining the evolution to the severe forms of the disease. Several mechanisms have been suggested for the enhancement of disease severity following a first infection by a distinct serotype, including deposition of immune complexes, cross-reaction of antibodies with the vascular endothelium, infection intensification mediated by antibodies, activation of the complement system and its products, exacerbated release of soluble mediators, such as cytokines (Chatuverdi, *et al.,* 2000; Lin, *et al.,* 2005; Halstead, 1979; Halstead, 1989; Morens, 1994; Theofipoulos *et al.,* 1976; Bokisch *et al.,* 1973; Malasit, 1987; Chungue, *et al.,* 1994, Falconar, 1997; Markoff, *et al.,* 1991; Libraty, *eet al,* 2002; Kurane, *et al.,* 1994; Mangada, *et al.,* 2004).

Other studies associate factors, including age, genetics and host nutritional status, to disease severity; although mechanisms accounting for disease severity have not been investigated (Rothman, 2003). Therefore, a better knowledge of the pathogenesis of dengue infection and disease is indispensable in the search for specific and effective treatments.

Studies using an experimental model for dengue - which mimics several aspects of the severe form, including lethality, tissue hemorrhage, thrombocytopenia and hemoconcentration - were employed in the discovery of a novel use of receptor antagonists of PAF, described in the present invention.

In particular, the present invention relates to the use of a platelet activating factor receptor antagonist for the preparation of a medicament for treating infectious diseases caused by viruses of the Flaviviridae family, according to claim 1.

An analysis intends to contribute to show that physiopathological manifestations of dengue hemorrhagic fever (anomalous immune response with increased vascular permeability, increased hematocrit, lowered blood pressure, hypovolemic shock and thrombocytopenia) are very similar to those observed in septic shock (Halstead, 2003; Rothman, 2003; Sommers, M.S. (2003). The cellular basis of septic shock. Crit. Care Nurs Clin. North. Am. 15(1):13-25). Therefore, investigating the role of mediators involved in syndromes of systemic inflammatory response occurring in the pathogenesis of the dengue infection is pertinent.

Among such mediators, platelet activating factor (PAF) should be highlighted. The function of PAF can be blocked by the use of compounds which antagonize the receptor for PAF (PAFR). In addition to these PAFR antagonists, the function of PAF can be prevented by the administration of a recombinant enzyme called PAF acetylhydrolysis. The enzyme metabolizes PAF, thus preventing the binding of metabolized PAF to its PAFR.

Among the major effects of the systemic administration of PAF are vasodilatation and increased vascular permeability which results in hypotension and thrombocytopenia. These are findings which are similar to those found in dengue hemorrhagic fever. Furthermore, a previous study showed that PAF was released during dengue infection of human macrophages, hence suggesting a putative role of the mediator in the human disease (Yang KD, Lee CS, Shaio MF. A higher production of platelet activating factor in ex vivo heterologously secondary dengue-2 virus infections. Acta Microbiol Immunol Hung. 1995;42(4):403-7).

The presence of thrombocytopenia is commonly found in patients infected by the Dengue virus. In fact, some studies indicate that 70% of all infected patients present thrombocytopenia during the illness. Patients with the lowest platelet counts seem to be at higher risk of developing the more severe forms of the disease. The treatment with the receptor antagonist significantly blocked the development of thrombocytopenia and hemoconcentration in infected animals. The treatment with PAF receptor antagonist also prevented the lethality following experimental infection by the dengue virus in a very significant way. These data suggest that treating with PAF receptor antagonist may be useful for the treatment of all those infected individuals, viewing to prevent more severe forms of dengue infection.

Finally, data show that treating with a PAF receptor antagonist - even after several days of infection - is able to prevent animal mortality and impede frequent clinical alterations, such as thrombocytopenia and hemoconcentration.

Such results give support to the involvement of PAF receptors in the pathogenesis of dengue infection and also support the involvement of inflammatory mediators in the disease. Among its various biological actions, PAF is known to induce platelet aggregation, and leukocyte and endothelial cell activation. These parameters are important features of the host response to dengue virus infection (Halstead, 2003; Rothman, 2003).

Therefore, for the present invention, experiments were carried out in order to corroborate the new intended use of PAFR antagonists for the treatment of dengue infection. Animals were treated with vehicle or a PAF receptor antagonist and infection and inflammatory parameters assessed, as is shown in the following examples.

### Example 1

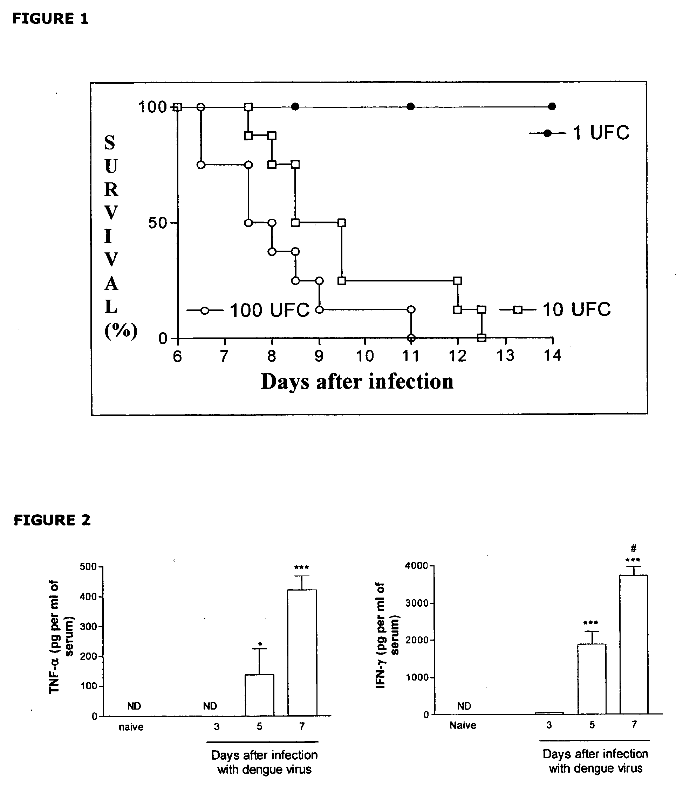

Laboratory studies have shown that mice infected with the dengue virus (dengue serotype-2, Access number at GeneBank AY927231) presented clinical manifestations and laboratory alterations resembling those found in patients having severe dengue forms, including hemorrhagic fever and the shock syndrome caused by dengue. As can be observed, there is death when mice receive increased inocula of viruses, which is dependent on the amount of virus inoculated (shown in Figure 1 as CFU - colony forming units).

### Example 2

Infection with the dengue virus causes thrombocytopenia and hemoconcentration (Figure 2), which are frequently found in patients with dengue, mainly in those who develop more serious forms of the disease.

### Example 3

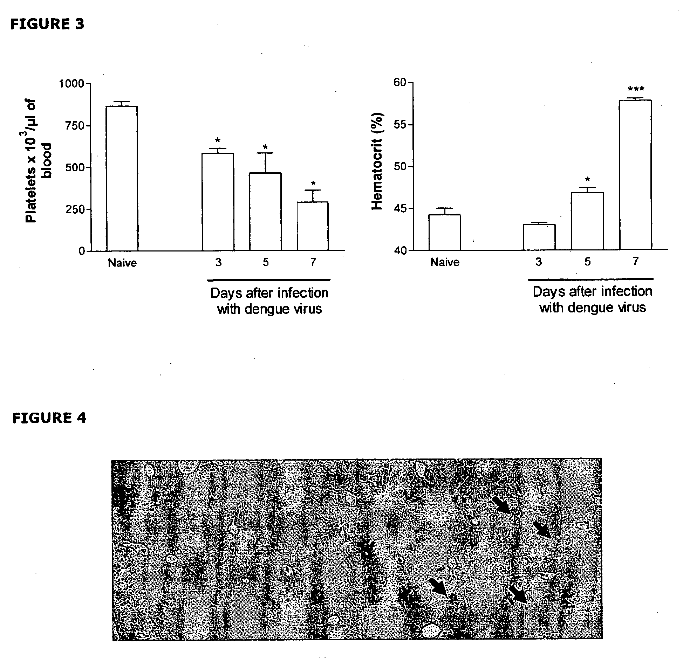

In addition to the manifestations described above, mice infected with dengue showed other alterations that are compatible with human infection, including increased levels of circulating cytokines, especially the cytokine called tumor necrosis factor-α (TNF-α) and interferon-γ (IFN-γ) (Figure 3).

### Example 4

There are also significant alterations in tissues of animals infected with the dengue virus, including in the liver of infected animals. In Figure 4, a large amount of blood cells is observed in the liver parenchyma (indicated by arrows) of infected animals, which is compatible with the bleeding observed in humans with the hemorrhagic dengue fever.

### Example 5

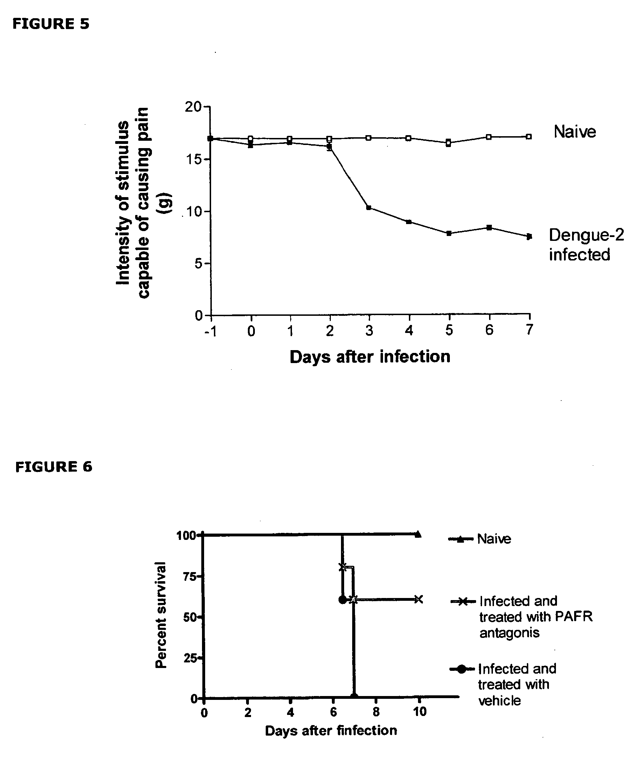

Mortality begins on day 7 or 8 after infection, when the immune response of the organism peaks. On the other hand, animals show perceptible disease symptoms form the third day after infection. Figure 5 shows that sensitivity of animals to a painful stimulus is significantly heightened which is correlated with the pain reported by humans having dengue fever. Dengue is widely known as "break-bone fever". Similarly, treating with the PAF receptor antagonist has partially prevented the death of animals infected by the dengue virus.

### Example 6

The animal model has been used in order to test the hypothesis that blockage by specific PAF receptor antagonists would be able to modify experimental infection by the dengue virus. Initial studies have shown that daily treatment with a PAF receptor antagonist (aPAFR) is able to prevent mortality induced by the virus. It has been significantly shown that the treatment with this PAF receptor antagonist initiated in days 3 (3-10) or 5 (5-10) after the infection onset was as efficient in preventing the death of animals as that initiated before infection (0-1). The treatment begun 7 days after infection (7-10), when some lethality was already observed, was partially effective in preventing death of animals. As a whole, the data show that treating with such a PAF receptor antagonist was able to prevent the death of infected animals not only when it was administered in a prophylactic way but also when administered therapeutically, i.e., after symptoms appeared (Figure 6). As can be seen in Figure 5, symptoms in animals.are notorious from the third day of the infection onset.

Despite the PAF receptor antagonist treatment having prevented mortality and more relevant clinical and pathologic alterations, viremia was not altered in vehicle- and PAFR antagonist-treated animals. In fact, data suggest that viremia was smaller in animals treated with the PAF receptor antagonist, which may be secondary to a better immunological response associated with a better general clinical status of animals.

### Example 7

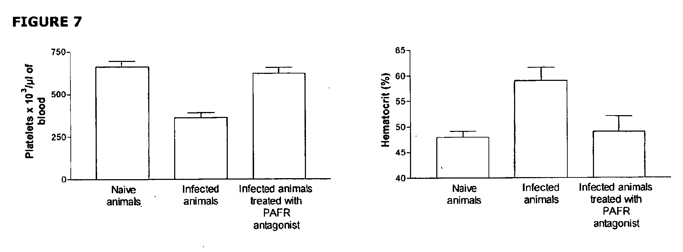

Animals treated with a PAF receptor antagonist presented milder clinical signs after being infected with the serotype 2 of dengue virus as compared to animals infected with the same inoculum and treated with vehicle. Graph in Figure 7 show a reduced number of platelets in infected animals and the prevention of platelet reduction in infected animals which received the PAF receptor antagonist. The Figure also shows an increase in hematocrit, a marker of hemoconcentration due to increased vascular permeability, in infected animals. The treatment with the PAFR antagonist also prevented changes in hemoconcentration.

### Brief Description Of The Figures

**FIGURE 1****:** Infection with Dengue virus induces a inoculum-dependent death of mice. Death is usuall observed after the 7th day of infection. The graph shows the number of surviving animals at the given days after infection.
**FIGURE 2****:** Infection with dengue virus induces an increase in the serum levels of the cytokines TNF-α and IFN-γ. Cytokines are first detected in plasma from day 5 after infection and peak at day 7, at the time death is first observed.
**FIGURE 3****:** Infection with dengue virus induces thrombocytopenia (fall in platelet numbers) in blood and increases hematocrit (a marker of hemoconcentration). Fall in platelet numbers is first observed at day 3 after infection, whereas hemoconcentrations is first detected at day 5.
**FIGURE 4****:** Hematoxylin and eosin-stained sections of liver from uninfected mice (left) and mice infected with dengue virus at day 7 after infection. Note the significant presence of red blood cells (arrows) in the tissue, an indication of hemorrhage. Also note areas of liver degeneration (faintly stained).
**FIGURE 5****:** Infection with dengue virus induces an increase in hypernociception, an experimental marker of pain, that is first observed at day 3 after infection. Hence, first symptoms due to the infection are first noticed at day 3.
**FIGURE 6****:** Treatment with a PAFR antagonist prevents death associated with infection with dengue virus. The graph shows the number of surviving animals at the given days after infection.
**FIGURE 7****:** Treatment with a PAFR antagonist prevents the thrombocytopenia (fall in platelet numbers) and hemoconcentration (increase in hematocrit) observed at day 7 in dengue-infected animals.

## Claims

1. Use of a platelet activating factor receptor antagonist for the preparation of a medicament for treating infectious diseases caused by viruses of the *Flaviviridae* family.

2. Use according to claim 1 wherein said infectious diseases caused by viruses of the *Flaviviridae* family is Dengue.

3. Use according to claim 1 wherein said infectious diseases caused by viruses of the *Flaviviridae* family is Dengue Hemorrhagic Fever or Shock Syndrome caused by Dengue.

4. Use according to claim 1 wherein said medicament is to be administered to mammals.

5. Use according to claim 1 wherein said medicament is to be given via endovenous, intramuscular, oral, subcutaneous, transdermal or intraperitoneal administration.

6. Use according to claim 1 wherein said platelet activating factor receptor antagonist is liable to be selected from the following substances UK-74505, SDZ 64-412, SDZ 64-688, SR27417A, Y-24180, WEB2086, WEB2170, BN50730, BN52501, SM-10661, PCA-4246, ABT-491, BB-882 (lexipafant) and TCV-309.

7. Use according to claim 1 wherein said medicament can be administered together with other pharmaceuticals for treating infections caused by viruses from the *Flaviviridae* group.

## Patentansprüche

1. Verwendung eines Thrombozyten aktivierenden Faktor-Rezeptor-Antagonisten für die Herstellung eines Medikamentes zum Behandeln von Infektionserkrankungen, verursacht durch Viren der Flaviviridae-Familie.

2. Verwendung gemäß Anspruch 1, wobei die Infektionserkrankung, welche durch Viren der Flaviviridae-Familie verursacht werden, Dengue ist.

3. Verwendung nach Anspruch 1, wobei die Infektionserkrankungen, welche durch Viren der Flaviviridae-Familie verursacht werden, hämorrhagisches Denguefieber oder durch Dengue verursachtes Schocksyndrom ist.

4. Verwendung nach Anspruch 1, wobei das Medikament Säugern verabreicht wird.

5. Verwendung nach Anspruch 1, wobei das Medikament intravenös, intramuskulär, oral, subkutan, transdermal oder intraperitoneal verabreicht wird.

6. Verwendung nach Anspruch 1, wobei der Thrombozyten aktivierende Faktor-Rezeptor-Antagonist ausgewählt wird aus den folgenden Substanzen: UK-74505, SDZ 64-412, SDZ 64-688, SR27417A, Y-24180, WEB2086, WEB2170, BN50730, BN52501, SM-10661, PCA-4246, ABT-491, BB-882 (lexipafant) und TCV-309.

7. Verwendung nach Anspruch 1, wobei das Medikament zusammen mit weiteren Pharmaka zum Behandeln von Infektionserkrankungen, die durch die Flaviviridae-Gruppe verursacht werden, verabreicht wird.

## Revendications

1. Utilisation d'un antagoniste du récepteur du facteur d'activation plaquettaire pour la préparation d'un médicament pour traiter des maladies infectieuses provoquées par des virus de la famille des *Flaviviridae.*

2. Utilisation selon la revendication 1, dans laquelle lesdites maladies infectieuses provoquées par des virus de la famille des *Flaviviridae* sont la dengue.

3. Utilisation selon la revendication 1, dans laquelle lesdites maladies infectieuses provoquées par des virus de la famille des *Flaviviridae* sont la fièvre hémorragique de la dengue ou un syndrome de choc provoqué par la dengue.

4. Utilisation selon la revendication 1, dans laquelle ledit médicament est destiné à être administré à des mammifères.

5. Utilisation selon la revendication 1, dans laquelle ledit médicament est destiné à être administré par administration endoveineuse, intramusculaire, orale, sous-cutanée, transdermique ou intrapéritonéale.

6. Utilisation selon la revendication 1, dans laquelle ledit antagoniste du récepteur du facteur d'activation plaquettaire peut être sélectionné parmi les substances suivantes : UK-74505, SDZ 64-412, SDZ 64-688, SR27417A, Y-24180, WEB2086, WEB2170, BN50730, BN52501, SM-10661, PCA-4246, ABT-491, BB-882 (lexipafant) et TCV-309.

7. Utilisation selon la revendication 1, dans laquelle ledit médicament peut être administré conjointement avec d'autres produits pharmaceutiques pour traiter des infections provoquées par des virus du groupe des *Flaviviridae.*
